# EUROPEAN PATENT APPLICATION

(11) **EP 2 238 995 A1**
(43) Date of publication of application: **13.10.2010**
(21) Application number: 09005223.4
(22) Date of filing: 09.04.2009
(51) Int. Cl.: A61M 1/34

(54) **System for the treatment of blood**

(71) Applicant: Gazzano, Michele, 20090 Trezzano Sul Naviglio (MI) (IT)
(72) Inventor: Farnikova, Helena, 93049 Regensburg (DE)
(74) Representative: Barberi, Vittorio

(57) **Abstract**

The present invention relates to a system for the treatment of blood, in particular a highly efficient system for the safe removal of CO2 and bicarbonates from the blood in a blood circuit connected to a patient by means of a venous-venous connection having at least an inlet or tube line (1) for receiving a flow of blood for CO2 (or bicarbonate) removal and an outlet or tube line (9) for the blood deprived of CO2 (or bicarbonate); the system comprises: - an hemofilter (or dialyzer) (3), disposed between said inlet (1) and said outlet (9), and provided with means for separating the plasmatic water from the blood and for conveying the same plasmatic water thru a second tube line (4); - a device (6) for removing bicarbonates and/or CO2, disposed and acting on said second tube line (4), in a position which is downstream of said hemofilter (3) and upstream of a third tube line (8) which brings back the treated plasmatic water in the blood circuit downstream of said hemofilter (3).

## Description

The present invention relates to a system for the treatment of blood. In particular, the invention concerns a highly efficient system for the safe removal of CO2 and bicarbonates from the blood.

During these last years the medical community recognized that Mechanical Ventilation in Intensive Care Units is a necessary but dangerous practice; in fact, even if often represents the only possibility to maintain the patient alive, it brings high risks and, if no properly settled, it is often the cause of Ventilatory Induced Lung Injury (VILI), Barotraumas, Pulmonary Fibrosis, etc.

For this reason some tentative solutions for patients with damaged lungs arose within the extracorporeal approach: the gas exchange, that usually occurs in the Alveoli, is obtained by means of a circuit in which the patient's blood meets a supplied oxygen flow in a component usually called oxygenator; in said component, for the differences in relative partial pressures, oxygen reaches the red cells in the blood stream and the CO2, that comes from the metabolic production, goes out. This approach would greatly help the correct setting of the ventilator machine within very protective parameters and, in some cases, it avoids the use of the Mechanical Invasive Ventilation allowing the patient to be treated with Non Invasive Ventilation or even with only spontaneous breathing.

Several different approaches have been experimented.

A first attempt employs the difference in pressure between the arteries and veins to get the blood pass thru the oxygenator; but, since the arterial blood is relatively poorer in CO2 content and richer in 02, it is necessary an high flow to get the complete removal of CO2. To get this flow, a relatively large catheter must be inserted in the patient' vessels, both in an artery and in a vein (two accesses). The arterial blood that goes thru the oxygenator do not pass the natural regions of the body that therefore lack a proper blood supply. This solution generates a risk of ischemia of lower limbs. Furthermore, it naturally requires an arterial access (with risk of hemorrhage and contamination), needs a stable (or pharmacologically stabilized) arterial pressure and cardiac output. Other systems use a venous-venous approach, thus requiring a pump driven system. This approach is easier and safer because there is no need for inserting a catheter in an artery. The treatment can be even less invasive reducing the blood flow below 500 ml/min, this often requires only one venous access point (with a double lumen catheter), with the only disadvantage represented by a reduced efficiency but without any dangerous side effects. At the moment, these mini-invasive systems do not exceed the removal of 50% of the metabolic production in CO2 and an improvement of this removal capability would be very advantageous in a extended area of application therapies.

It is an aim of the present invention to provide a system for a greater removal of CO2 and bicarbonates from the blood, in a safe way, acting on (but not limited to) the plasmatic water obtained from the blood. In other words, the CO2 and the bicarbonates are not removed directly from the whole blood, but from the plasmatic water which has been previously separated from the other more fragile components of the blood thanks to a Hemofilter (or similar device). This solution gives the possibility to treat only the fraction of blood that contains high percentage of bicarbonates with procedures that would damage the other components (red cells, white cells, platelets, etc.)
For a better comprehension of the new technique here described, it will be explained how gases are carried in the blood circulation.

Oxygen (02) is linked to hemoglobin, a protein inside the red cells. The quantity of oxygen that a certain amount of blood can carry is fixed: four molecules of oxygen per each molecule of hemoglobin. Under normal condition 100 ml of blood can carry 20.1 ml of oxygen (02).

A different system is used for the transport of CO2: a percentage of about 70% (different studies give different values, these values must be considered as averages for explanation purposes) is converted in bicarbonate ions (HCO₃⁻) the bicarbonates are a sort of "concentrated" form of CO2 , about the 25% is carried as carbamine compounds, where CO2 is linked with proteins inside the red cells. Approx. the 5% is free, in solution.

The bicarbonate ions are in solution in the plasmatic water, outside the solid components of blood. The plasmatic water can be easily separated from blood with a standard hemofilter (for Dialysis or for C.R.R.T. Continuous Renal Replacement Therapy).

According to the present invention, the plasmatic water (also called ultrafiltrate) can be submitted to treatment that would damage the other blood components in whole blood; these treatments, which can be different and in combination of two or more, can reduce dramatically the content of bicarbonate ions thus reducing the total content of CO2 in the blood without any side effect on white and red cells.

After the treatment, the plasmatic water with reduced bicarbonate content is brought back and mixed with the blood flow before giving it back to the patient.

In this way, even with an extracorporeal circuit with a blood flow of less than 500 ml/min, it is possible to remove a bicarbonates content that corresponds to the total production of metabolic CO2, relieving the damaged lungs from the task of exchanging waste products.

According to the present invention, there is a system as claimed in Claim 1. Other features of the invention are described in the dependant claims.

The advantages of the invention will be more evident by the following description.

A non-limiting embodiment of the present invention will be described by way of example with reference to the accompanying drawings, in which:
- Figure 1 shows, schematically, a circuit relevant to a system for the treatment of the blood in accordance with the teachings of the present invention;
- Figure 2 shows another possible embodiment of the invention;
- Figure 3 shows a further embodiment of the invention;
- Figure 4 shows a possible embodiment of a component of the circuits shown in the Figures 1-3;
- Figures 5, 6, 7 show, schematically, three further embodiments of the invention.

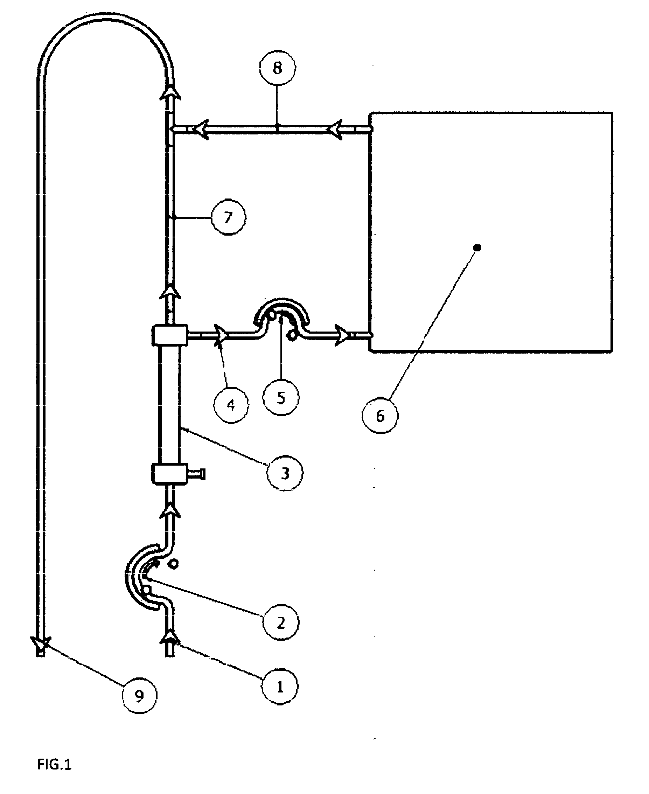

With reference to the enclosed drawings, the connection to the patient is of venous-venous type. In practice, the blood comes from and returns to a patient vein. A possible embodiment of a circuit in accordance with the invention has been explained referring to Figure 1.

Using the reference numerals of the drawings, the blood comes from the patient's vein, usually the Jugular or the Femoral, by a tube line (1) drained by means of a first pump (2).

Downstream of the pump (2) is disposed a Hemofilter or a Dialyzer (3), in which is pushed the blood for separating the Plasmatic Water (which, downstream of the Hemofilter 3, passes in the tube line 4) and the solid components of blood (which, downstream of the Hemofilter 3, pass in the tube line 7).

The Plasmatic Water, which passes in the tube line (4), is drained by the pump (5), generally at a flow that is not greater than the 25% of the blood flow to avoid excess of fluidity loss in the remaining blood.

Downstream of the tube line (4) there is a device for removing bicarbonates and/or CO2, schematically represented by the block (6). In particular, the block (6) represents one (or a combination of more) of the possible methods for removing bicarbonates and/or CO2 which will be described below,.

Downstream of the block (6), the treated Plasmatic Water is brought back to the main fraction of blood by a tube line (8), thus coming back to the original formula except for a great reduction in CO2/bicarbonates content. This blood is given back to the patient with the tube line (9).

According to the invention, it is very important the advantage of the venous-venous approach in terms of safety for the patient, easiness of treatment management and great reductions in side effects. The aim of the invention is to remove with a single catheter (double lumen) and with a low flow, the amount of the CO2 metabolic production close to the 100%.

This simple circuit has the added advantage of a low cost components set.

Advantageously, in order to achieve better results it will be possible to add, before or after the hemofilter of the circuit of Figure 1, an oxygenator which may help in removing the fraction of CO2 that is linked to proteins (25 - 30%) inside the red cells and the small percentage that is in solution (5-10%).

The presence of the Oxygenator (10) will also give a contribution to the patient's damaged lungs with the complete oxygenation of the portion of blood treated. The effect of the Oxygenator will also greatly compensates the pH modification produced by the bicarbonate removal bringing it back closer to the standard values.

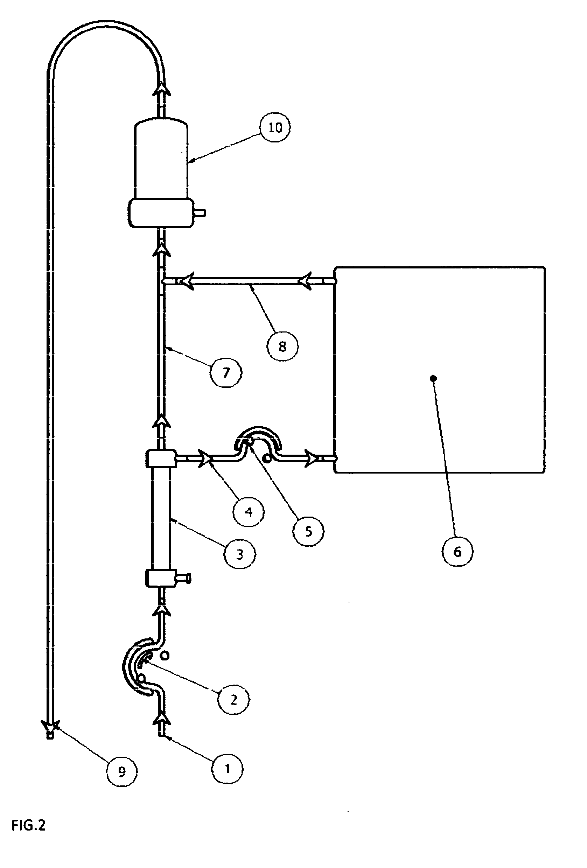

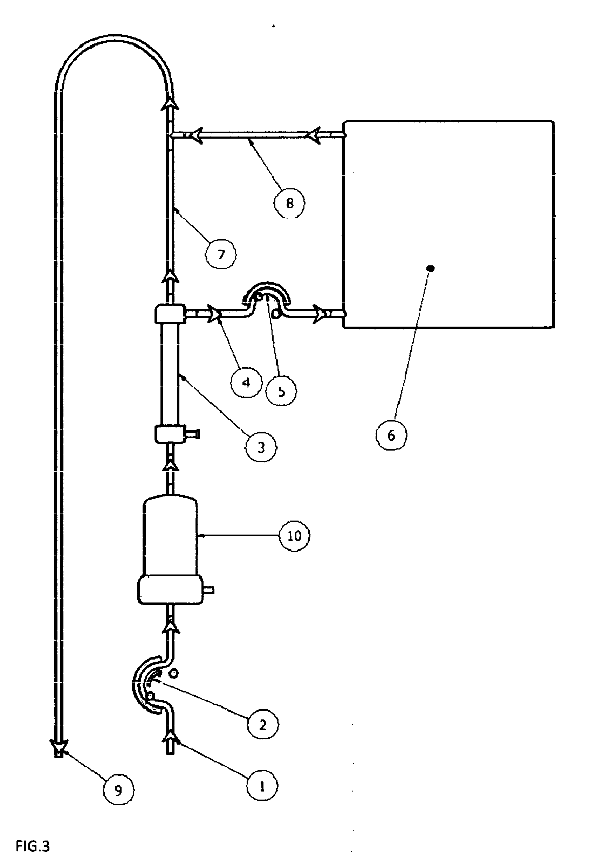

Two possible embodiments of a circuit including the oxygenator are shown in Figures 2 and 3.

In the example of Figure 2 the oxygenator (10) is disposed downstream of the Hemofilter (3) and downstream of the tube line (8), i.e. in a zone in which the purified plasmatic water is brought back to the untouched fraction of the blood. In this way, it is possible to increase the quantity of bicarbonate and CO2 removed.
in the example of Figure 3 the oxygenator (10) is disposed upstream of the Hemofilter (3). In this way, it is possible to obtain downstream of the same oxygenator, a blood (and a plasmatic water) having a fully percentage of oxygen.

The presence of oxygen may vary the equilibrium in the reaction that transforms CO2 in bicarbonates, in carbonic acid (that is a middle product) and in solution; thus, the choice of the best circuit will be influenced by this factor. In this position, furthermore, the blood pressure inside the Oxygenator will be increased by the resistance of the hemofilter to the blood flow, thus reducing the risk of direct passage of 02 in bubbles in the blood stream, that could bring to embolisms danger.

The block (6) represents one or more of the following sub-systems:

### A) Osmosis

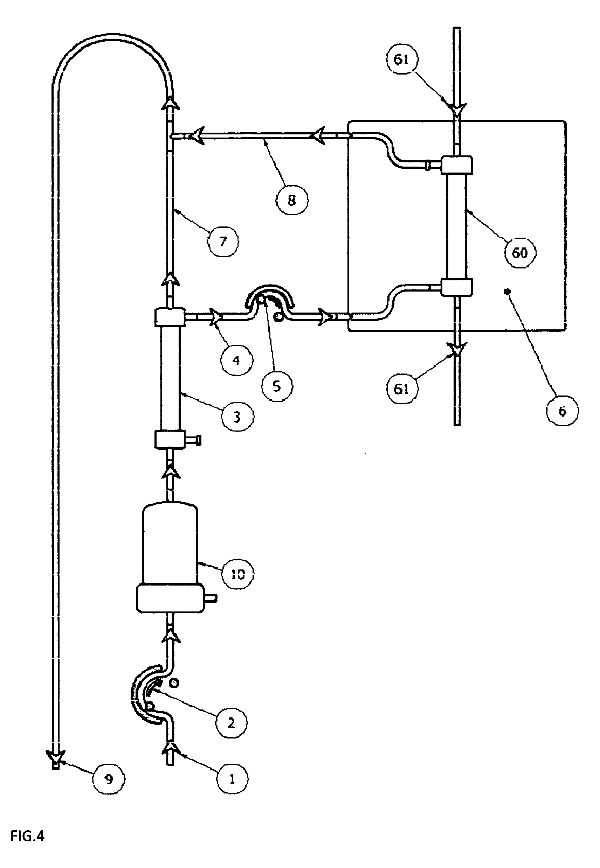

The plasmatic water is pushed in a device where an osmotic membrane separates it from a similar solution (like dialysis solutions) with less or no bicarbonates. For the osmotic effect, bicarbonates migrates thru the membrane and goes in the other solution, leaving the Plasmatic Water. This result can be easily obtained making the Plasmatic Water to pass thru a dialyzer filter (61) where the other side is washed with the proper solution. In Figure 4 is shown a possible example in which said similar solution passes thru the tube line (61), which, in turn, passes thru the dialyzer filter (60).

### B) Heat

The Plasmatic Water is heated to a convenient temperature (about 90 °C). At this temperature the bicarbonates precipitate; the Plasmatic Water passes a standard convenient filter that holds the precipitate and after being cooled at 40°, it is mixed back with blood (this will also reduce the loss of temperature in the extra corporeal circuit as an added advantage). According to this example, the system comprises an heating device acting on the plasmatic water; in practice, the block (6) comprises a duct and/or a filter for the passage of the plasmatic water and one or more heating means apt to heat the same plasmatic water.

### C) Electrolysis

1) The plasmatic water goes under an electrolytic cell where positive H+ ions are delivered by an electrode. The bicarbonates in the plasmatic water will transform to CO2 increasing the CO2 partial pressure that can be eliminated in a standard bubble-trap chamber or in an oxygenator.
2) The proper current and electrodes will catalyze bicarbonate into the electrolytic cell, removing it from the water circulation and blood.
   In this case, the system includes: an electrolytic cell (or similar device), means for eliminating the reaction products (filter).

### D) Precipitation

A container, filled with the proper material, (i.e. Calcium Gluconate) will make the bicarbonate in the Plasmatic Water to precipitate. A convenient filter will hold it inside the container. In this case, the system includes a container filled with a precipitating substance which determine the precipitation of the bicarbonate, filtering means for collecting the residual products.

### F) Chemical transformation

The plasmatic water will pass thru a container filled with Barium Hydroxide pellets that would react with bicarbonate forming Barium Carbonate, water and hydroxyl ions. Other chemical products could be used with the same objective, i.e. for forming a Carbonate reacting with the bicarbonate contained in the plasmatic water. In this case, the system includes a container filled with Barium Hydroxide pellets or other similar chemical products.

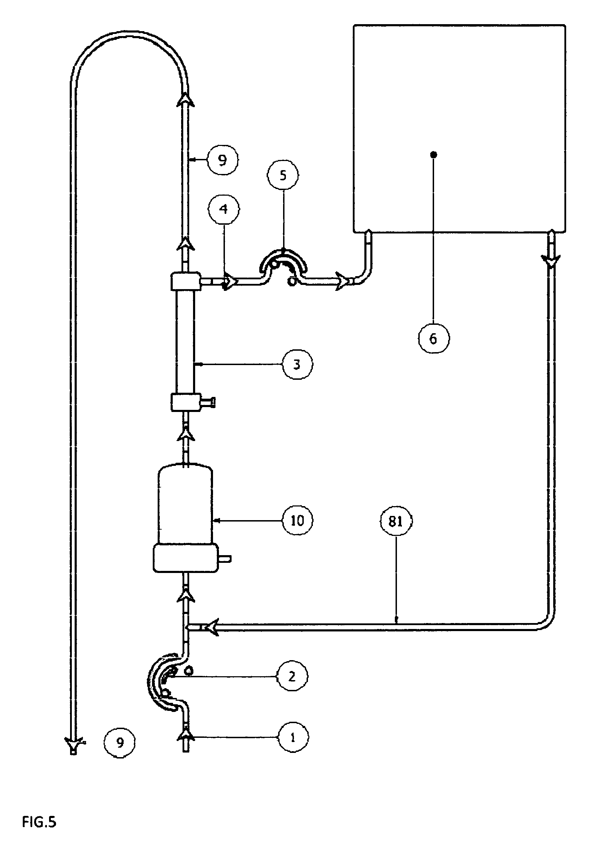

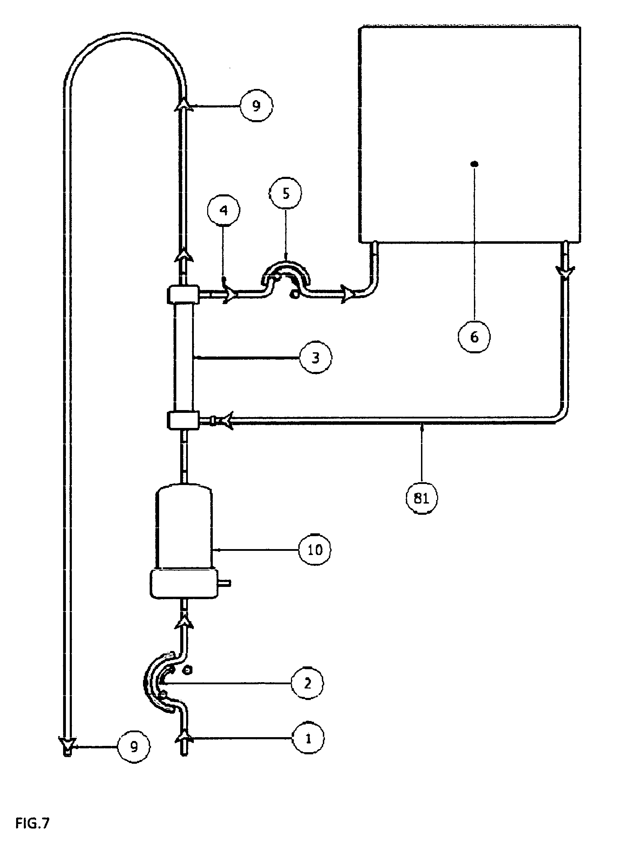

In the embodiments of the invention shown in the drawings of Figures 5-7, the output of the device (6) is connected to the blood circuit upstream of the hemofilter (3) or in correspondence of the same hemofilter (Figure 7).

In these cases the system comprises an oxygenator.

According to the example of Fig.5, a third line tube (81) connects the device (6) to the blood circuit upstream of the oxygenator (10), which, in turn, is disposed upstream of the hemofilter (3) and downstream of the pump (2). In practice, the third line tube (8) is connected to the blood circuit in a point disposed between the pump (2) and the oxygenator (10).

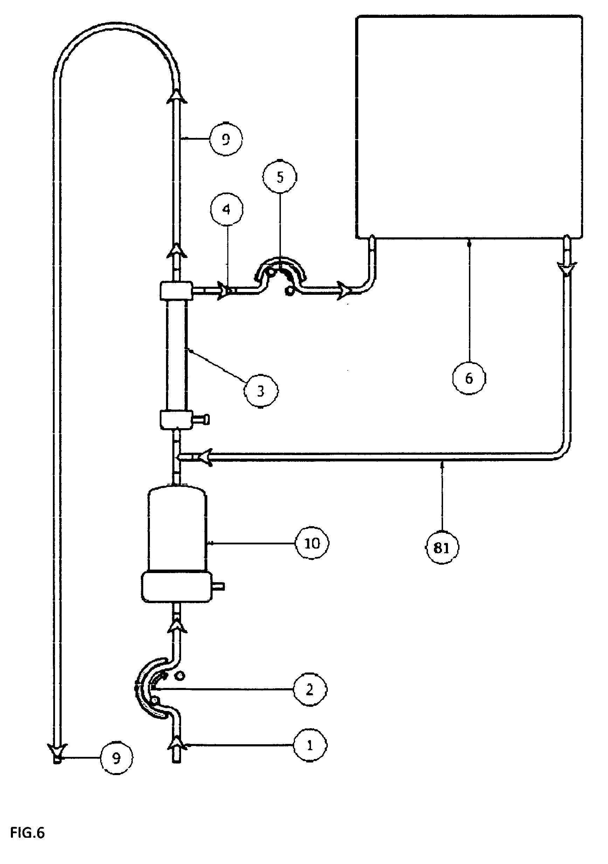

In the example of Fig.6, the third line tube (81) connects the device (6) to the blood circuit upstream of the hemofilter (3) and downstream of the oxygenator (10).

In the example of Fig.7, the third line tube (81) connects the device (6) to the blood circuit directly to one of the inlets of the plasmatic water side into the hemofilter (3); in practice, the tube line (8) is connected to the upstream inlet of the hemofilter (3) which in the other examples is closed and not used. In this way the plasmatic water recirculates and becomes the mean for the osmotic removal of bicarbonates and / or CO2.

The system will also include the possibility for injection of anticoagulants (like Heparin or Calcium-Citrate solutions as commonly used during standard extracorporeal treatments.

Clearly, changes may be made to the blood treatment machine and unit as described and illustrated herein without, however, departing from the scope of the present invention.

## Claims

1. System for the treatment of blood, in particular for the safe removal of CO2 and Bicarbonates from the blood, in a blood circuit connected to a patient by means of a venous-venous connection having at least an inlet or tube line (1) for receiving a flow of blood for CO2 (or bicarbonate) removal and an outlet or tube line (9) for the blood deprived of CO2 (or bicarbonate), **characterized in that** it comprises:
an hemofilter (or dialyzer) (3), disposed between said inlet (1) and said outlet (9), and provided with means for separating the plasmatic water from the blood and for conveying the same plasmatic water thru a second tube line (4);
a device (6) for removing bicarbonates and/or CO2, disposed and acting on said second tube line (4), in a position which is downstream of said hemofilter (3) and upstream of a third tube line (8) which brings back the treated plasmatic water in the blood circuit downstream of said hemofilter (3).

2. System according to claim 1, **characterized in that** said device (6) comprises an osmotic membrane which, for the osmotic effect, determinates the migration of the bicarbonates thru the membrane, leaving the Plasmatic Water.

3. System according to claim 1, **characterized in that** said device (6) comprises an heating device acting on the plasmatic water.

4. System according to claim 1, **characterized in that** said device (6) comprises: an electrolytic cell and means for eliminating the relevant gas or precipitates formed by the reaction with the plasmatic water.

5. System according to claim 1, **characterized in that** said device (6) comprises a container filled with a precipitating substance which determine the precipitation of the bicarbonate, filtering means for collecting the residual products.

6. System according to claim 1, **characterized in that** said device (6) comprises a container filled with a Barium Hydroxide pellets or with another similar substance suitable for reacting with bicarbonate contained in plasmatic water.

7. System according to one of the preceding claims, **characterized in that** said device (6) comprises an oxygenator (10) disposed upstream of the hemofilter (3).

8. System according to one of the preceding claims from 1 to 6, **characterized in that** said device (6) comprises an oxygenator (10) disposed downstream of the hemofilter (3).

9. System for the treatment of blood, in particular for the safe removal of CO2 and Bicarbonates from the blood, in a blood circuit connected to a patient by means of a venous-venous connection having at least an inlet or tube line (1) for receiving a flow of blood for CO2 (or bicarbonate) removal and an outlet or tube line (9) for the blood deprived of CO2 (or bicarbonate), **characterized in that** it comprises:
- an hemofilter (or dialyzer) (3), disposed between said inlet (1) and said outlet (9), and provided with means for separating the plasmatic water from the blood and for conveying the same plasmatic water thru a second tube line (4);
- an oxygenator (10) disposed upstream of the hemofilter (3);
- a device (6) for removing bicarbonates and/or CO2, disposed and acting on said second tube line (4), in a position which is downstream of said hemofilter (3);
- a third tube line (81) which brings back the treated plasmatic water in the blood circuit upstream of said hemofilter or in an inlet of the same hemofilter (3) disposed upstream.

10. System according to claim 9, **characterized in that** third tube line (8) is connected to the blood circuit upstream of the hemofilter (3) and downstream of the oxygenator (10).

11. System according to claim 9, **characterized in that** third tube line (8) is connected to the blood circuit upstream of the hemofilter (3) and of the oxygenator (10).

12. System according to one of the preceding claims, **characterized in that** said device (6) comprises a first pump (2) disposed upstream of the hemofilter (3).

13. System according to one of the preceding claims, **characterized in that** said device (6) comprises a second pump (5) disposed on said second tube line (4).
